# EUROPEAN PATENT SPECIFICATION

(11) **EP 0 642 801 B1**
(45) Date of publication and mention of the grant of the patent: **29.04.1998**
(21) Application number: 94114260.6
(22) Date of filing: 10.09.1994
(51) Int. Cl.: A61M 1/36

(54) **Disposable sterile apparatus for blood filtration with system for optimizing the recovery of blood between pouches**
Sterile Blutfiltrationsvorrichtung mit einem System zum Optimieren der Blutrückgewinnung
Dispositif stérile pour filtration du sang avec système d'optimisation de récupération du sang

(30) Priority: 15.09.1993 IT MI931992
(43) Date of publication of application: 15.03.1995
(73) Proprietor: BIOFIL S.r.l., I-41036 Medolla (MO) (IT)
(72) Inventor: Mari, Giorgio, Dr., I-41037 Mirandola (MO) (IT)
(74) Representative: Luderschmidt, Schüler & Partner GbR

(56) References cited:
- WO-A-91/17809
- DE-A- 3 012 110
- FR-A- 2 695 037

## Description

The present invention concerns a disposable sterile apparatus for the filtration of blood with a system for optimizing the recovery of blood in the passage from one container (pouch) to another.

The use of pre-assembled lines, designated tube sets, for single use to assure the sterility of the assembly for the filtration of blood contained in one pouch to a second empty pouch is now consolidated in the medical equipment sector.

The foregoing sets are essentially composed of a tube in plastic equipped at the ends with quick couplings for a spike and a pouch that is empty of both liquid and air. At the center of the section of tube there is a filter designed to filter out any leukocytes contained in conserved blood and platelet concentrates, extraneous particles, etc.

During use the set is connected to a blood container, which is normally a pouch having the same dimensions and characteristics as the empty pouch, said container being suspended from a stand and, due to the effects of gravity, the medicament or the blood drips into the empty pouch.

At the end of the process, i.e. when the upper container is empty, the final volume of liquid or blood present in the tube set tends to remain in the tube because of the negative pressure progressively created inside the assembly; because of this phenomenon a final quantity of blood or medical liquid is effectively lost during the filtration process.

To solve this problem the Applicant has already developed a perfected tube set, which formed the subject of Italian patent publication no. JT00225396 disclosing a set that has a branch in the main tube in the proximity of the container, the extremity of which is connected to a filter in communication with free outside air and is therefore able to restore atmosperic pressure in the central section of the system, thus making it possible to evacuate and hence utilize the liquid or the blood present in this section.

Notwithstanding this invention, because of the characteristic microporosity of the median filter element, and particularly, because after the filtration of blood the filter element tends to clog progressively thus reducing its degree of porosity in such a way that free air is no longer able to enter the filter medium and therefore complete emptying of the pouch is prevented.

In practical terms, the so-called "bubble point" of the filter, this being the pressure value that the air must reach in order to pass through the capillaries of the filtration element soaked in blood, is not effectively reached so that the filter remains saturated.

To solve this problem, the Applicant has already developed a perfected filter, which formed the subject of patent application no. MO92U000018, disclosing a filter, illustrated in figure 2, that comprises a filter body, having an inlet and an outlet, the filter being divided into at least two semi-chambers by an internal partition of filter material and one of these two chambers, the semi-chamber serving for evacuation being equipped with its own air inlet line in communication with the area external to the filter body.

These solutions, which have proved efficient, require an inlet filter to purify the outside air that is utilized to allow the filter to be completely evacuated.

Moreover, the air cleaned by the inlet filter will inevitably be less pure than the sterile air.

WO-A-9117809 discloses a sterile fluid processing system comprising a collection bag with conduits connecting it to a second container for receiving a fluid product. A functional biomedical device which may be a filter devided by filtration material into two semi-chambers is interposed between the containers In order to facilitate recovering of liquid trapped in various elements of the system, a gas inlet is provided upstream of the biomedical device and a gas outlet downstream of the device. For recovery of blood in the system gas retained in an air container is transferred into the gas inlet and gas is purged from the system through the gas outlet and is transferred to a second air container for holding air.

The purpose of the present invention is to eliminate the problems mentioned above by developing a disposable sterile apparatus for the filtration of blood with a system for optimization of the recovery of filtered blood, which serves to recover the blood remaining in the filter in an optimized manner with respect to known apparatuses and to do this in an completely sterile ambient.

Within the framework of the foregoing purpose, one scope of the present invention is to create an apparatus of simple design and high reliability that can be manufactured relatively easily and at competitive costs.

A second and equally important scope is to create an apparatus with the use of fewer elements than known devices.

The above scope, in conjunction with the forgoing purposes and other objects that will be outlined more clearly in the following pages, is achieved with the features of claim 1.

The characteristics and advantages of the invention will be clarified by the description of a preferred embodiment chosen for the purposes of illustration and shown in the drawing in which
- figure 1: is a front elevation of an apparatus according to the present invention;
- figure 2: is a cross section of a possible design of a filter utilized in the apparatus shown in figure 1.

With reference to the above-mentioned figures, 1 indicates an apparatus according to the present invention. 2 indicates a first container (pouch) containing the blood to be filtered. This first Container 2 is connected to the apparatus 1 by means of a spike 3 designed for insertion into an outlet of the first container 2.

The spike 3 is connected to a first initial section 4 of a main tube. On the first initial section 4 of the main tube there is a tube clamp 5 for starting the filtration. The first initial section 4 of the main tube is connected to a three-way union 6, which connects the first initial section 4 of the main tube to an air return tube 7 and a second initial section 8 of the main tube. The air return tube 7 includes a check valve 7a that allows the passage of air only toward the first container 2.

A second initial section 8 of the main tube is connected to a drip chamber 9a of an initial aggregate filter 9. On the second initial section 8 of the main tube there is a safety tube clamp 10. The outlet from said first filter 9 is connected to a front connector 11 of a second selective filter 12 by means of an intermediate section 13 of the main tube. One end of an evacuation tube 14 for emptying the second filter 12 is connected to the top of the drip chamber 9a beside the second initial section 8 of the main tube. The other end of the evacuation tube 14 is connected to a rear connector 15 of the filter 12. Inside the evacuation tube 14 there is a break-off element 14a that permits the passage of liquid through tube 14 only when said element 14a has been fractured by the user.

An outlet of the second filter 12 comprises an outlet connector 16 located at the bottom of the second filter 12 on the side of rear connector 15. Moreover, outlet 16 of the second filter 12 is connected to a second three-way union 17 by means of a first final section 18 of the main tube. On the first final section 18 of the main tube there is an air return tube clamp 18a. Moreover, the other end of the air return tube 7 is also connected to the three-way union 17.

Finally, an empty second container (pouch) 20 is connected to the outlet of union 17 by means of a second final section 19 of the main tube. The second final section 19 of the main tube includes a second safety tube clamp 19a.

It is emphasized that all the tubes in the apparatus disclosed in the invention will initially contain only sterile air and that the device is a "closed circuit system", that is, at no point during the process does external air enter the apparatus 1.

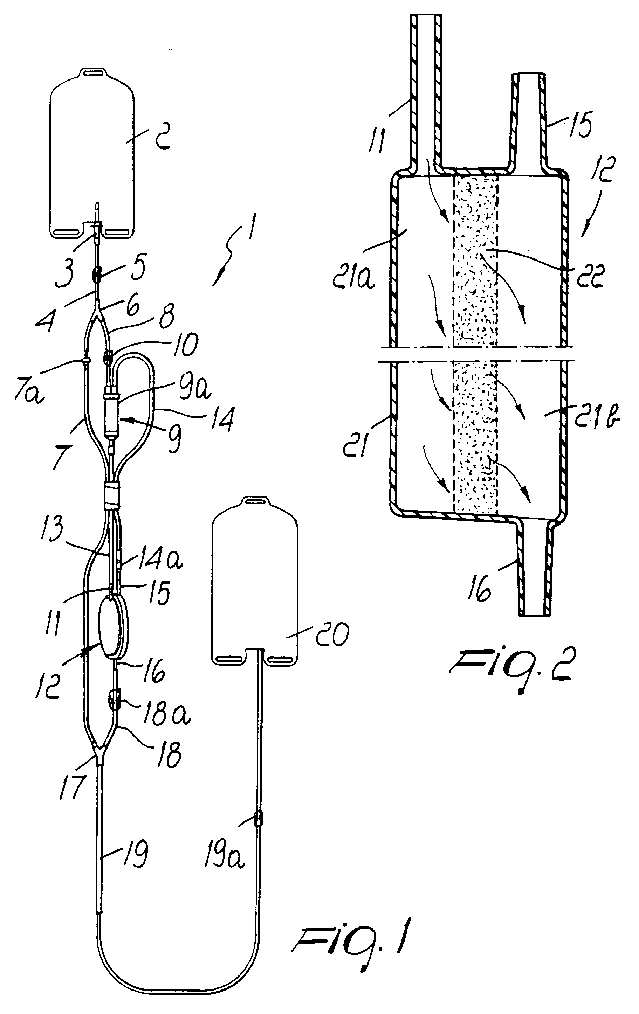

Figure 2 shows a possible design of the selective filter 12 which is the subject of patent application M092U000018. The filter 12 is essentially composed of a body 21 on which the front inlet connector 11 of the liquid to be filtered is located and also the rear connector 16 for emptying of the liquid to be filtered.

The filter body 21 is divided into at least two semi-chambers 21a and 21b which are, respectively, for the inlet of liquid to be filtered and the evacuation of the liquid after filtration, and a filter median partition 22. The front connector 15 serves to allow air to enter the evacuation semi-chamber 21b thus making it possible for the semi-chamber to empty completely.

Operation of the apparatus 1 in accordance with the claims of the present invention is as follows: The spike 3 is inserted into the first container 2 containing the liquid to be filtered. The filtration start tube clamp 5 is opened and the liquid to be filtered enters the first 4 and second 8 initial section of the main tube. The inlet of liquid into the air return tube 7 is prevented by the check valve 7a. The liquid continues to flow toward the first filter 9, dripping slowly into the drip chamber 9a. The inlet of liquid into evacuation tube 14 for emptying of the second filter 12 is prevented by the connection of said evacuation tube 14 to the top of the drip chamber 9a. If the drip chamber 9a should fill with liquid, the inlet to the evacuation tube 14 is equally prevented by the break-off element 14a.

After passing through the intermediate section 13 of the main tube the liquid enters the second selective filter 12 by way of the front inlet connector 11. The liquid passes through the filter partition 22 and filtered liquid exits from filter 12 through the rear outlet connector 16 thus filling the empty container 20 by way of the first 18 and the second 19 final section of the main tube.

When the first container 2 is emptied, sections 4, 8, 13, 18 and 19 of the main tube will remain full of liquid to be filtered because of the reduced pressure. Also second filter 12 will remain full of liquid to be filtered.

Once the first container 2 is empty, the second container 20 will contain the liquid from the first container 2 except for that liquid which has remained inside the second filter 12 and in the main tube sections 4, 8, 13, 18, 19 and the sterile air that was initially hold in the main tube sections 4, 8, 13, 18, 19 and in the two filters 9, 12, which has been forced into second filtered liquid container 20.

At this point the air return tube clamp 18a must be closed and the second container 20 must be turned upside down and pressed thereby forcing the sterile air which it contains to flow through the air return tube 7, the check valve 7a and the first section 4 of the main tube so that it enters the first container 2.

Once the air and the blood contained in the first initial section 4 of the main tube has passed from the second container 20 to the first container 2 the air return tube clamp 18a must be opened. The pressure of the air surrounding the liquid contained in the tube and in the filters (9, 12) is now equalized and, due to the force of gravity, the liquid empties from the main tube (and container 2 - the liquid previously contained in section 4), the first filter 9 and the first semi-chamber 21a of second filter 12, descending toward the second container 20.

At this point the break-off element 14a is fractured thereby permitting air to pass through evacuation tube 14. The air remaining in the first container 2 is sufficient to empty the evacuation semi-chamber 21b of the second filter 12.

Since the air is free to pass through both tubes, the intermediate section 13 of the main tube and the evacuation tube 14 of the second of filter 12, both the semi-chambers 21a and 21b of the second filter 12 are emptied, thereby further improving the recovery of filtered liquid.

The passage of air from one container to the other and the successive emptying of the tubes and the filters can, if necessary, be repeated several times.

It is also possible to compress the first container 2 to force the liquid transfer to the second container 20, even though this action is not strictly necessary since the effect of gravity is sufficient to recover liquid. Compressing the first container 2 is not recommended however, particularly when filtering blood, because the compression can give rise to modification of the corpuscular part of the blood.

While the invention has been described by reference to one preferred embodiment chosen for the purposes of illustration, it should be understood that numerous changes could be made to details of the design without departing from the scope of the invention as defined in the appended claims.

## Claims

1. A disposable sterile apparatus for blood filtration with a system for optimizing the recovery of blood comprising
a main tube (4, 8, 13, 18, 19) containing sterile air, one end of which can be connected to a first container (2) containing the liquid to be filtered and the other of which can be connected to an empty second container (20),
at least one filter (12) disposed along said main tube (4, 8, 13, 18, 19), said filter comprising a filter body defining an interior chamber and a filtration material (22) partioning the interior of the chamber into two semi-chambers (21a, 21b), the first semi-chamber (21a) having an inlet port (11) for the inlet of liquid to be filtered and the second chamber (21b) having an outlet port (16) for the evacuation of filtered liquid and
at least one air return tube (7) for the return of air from said second container (20) to said first container (2) acting to equalize pressure of the liquid at the two extremities of said main tube (4, 8, 13, 18, 19) and in said at least one filter (12) after the liquid has been filtered,
**characterized in** that
said second semi-chamber (21b) of said at least one filter (12) comprises an air inlet (15) communicating with said one end of the main tube (4, 8, 13, 18, 19) by means of an evacuation tube (14) acting to ventilize said second semi-chamber after filtration.

2. Apparatus according to claim 1, characterized in that said air return tube (7) includes a check valve (7a) that allows the passage of fluid from said second container (20) to said first container (2) and prevents the passage of fluid from said first container (2) to said second container (20).

3. Apparatus according to claim 1 or 2, characterized in that the air outlet extremity of said air return tube (7) is connected to said main tube (4, 8, 13, 18, 19) at an initial connection point (6) interposed between said end of said main tube (4, 8, 13, 18, 19) which can be connected to said first container (2) and said at least one filter (12).

4. Apparatus according to one or more of the previous claims, characterized in that the air inlet extremity of said air return tube (7) is connected to said main tube (4, 8, 13, 18, 19) at a second connection point (17) interposed between said at least one filter (12) and said second container (20).

5. Apparatus according to one or more of the previous claims, characterized in that it comprises closing means (18a) for cutting off the flow of liquid in said main tube (4, 8, 13, 18, 19) interposed between said at least one filter (12) and said other end of the main tube, said closing means (18a) acting to prevent the passage of air toward said at least one filter (12) during the passage of air from said second container (20) to said first container (2).

6. Apparatus according to claim 5, characterized in that said closing means (18a) is interposed between said at least one filter (12) and said second connection point (17).

7. Apparatus according to one or more of the previous claims, characterized in that said evacuation tube (14) comprises a fragile device (14a) acting to prevent the passage of fluid into said evacuation tube (14) and also acting to permit the passage of fluid in said evacuation tube (14) after the frangible device has been fractured.

8. Apparatus according to one or more of the previous claims, characterized in that it comprises a drip filter (9) interposed between said extremity of said main tube (4, 8, 13, 18, 19) which can be connected to said first container (2) and said at least one filter (12), said drip filter (9) including a chamber to the upper wall of which there is connected said evacuation tube (14) and said main tube (4, 8, 13, 18, 19), the lower wall being in communication with said at least one filter (12).

9. Apparatus according to one of claims 3 to 7, characterized in that it comprises a drip filter (9) interposed between said initial connection point (6) and said at least one filter (12), said drip filter (9) including a chamber to the upper wall of which there is connected said evacuation tube (14) and said main tube (4, 8, 13, 18, 19), the lower wall being in communication with said at least one filter (12).

## Patentansprüche

1. Sterile Blutfiltrationsvorrichtung zum einmaligen Gebrauch mit einem Optimierungssystem zur Rückgewinnung von Blut, umfassend
einen Hauptschlauch (4, 8, 13, 18, 19), der sterile Luft enthält, und dessen eines Ende an einen ersten Behälter (2), der die zu filtrierende Flüssigkeit enthält, angeschlossen werden kann, und dessen anderes Ende an einen leeren zweiten Behälter (20) angeschlossen werden kann,
zumindest einen Filter (12), der entlang des Hauptschlauchs (4, 8, 13, 18, 19) angeordnet ist und einen, eine Innenkammer umschreibenden Filterkörper und ein Filtrationsmaterial (22) umfaßt, das das Kammerinnere in zwei Halbkammern (21a, 21b) unterteilt, wobei die erste Halbkammer (21a) eine Einlaßöffnung (11) zum Einlaß der zu filtrierenden Flüssigkeit besitzt, und die zweite Kammer (21b) eine Auslaßöffnung (16) zum Entleeren filtrierter Flüssigkeit aufweist, sowie
zumindest einen Luftrückführschlauch (7) zur Rückführung von Luft vom zweiten Behälter (20) zum ersten Behälter (2), der so wirkt, daß der Druck der Flüssigkeit an den beiden äußersten Enden des Hauptschlauchs (4, 8, 13, 18, 19) und in dem zumindest einen Filter (12) ausgeglichen wird, nachdem die Flüssigkeit filtriert wurde,
**dadurch gekennzeichnet, daß**
die zweite Halbkammer (21b) des zumindest einen Filters (12) einen Lufteinlaß (15) umfaßt, der mit dem einen Ende des Hauptschlauchs (4, 8, 13, 18, 19) mittels eines Entleerungsschlauchs (14) in Verbindung steht, der so wirkt, daß die zweite Halbkammer nach Filtration belüftet wird.

2. Vorrichtung gemäß Anspruch 1, dadurch gekennzeichnet, daß der Rückführungsschlauch (7) ein Regulierventil (7a) umfaßt, welches den Flüssigkeitsdurchgang von dem zweiten Behälter (20) zum ersten Behälter (2) ermöglicht und den Flüssigkeitsdurchtritt vom ersten Behälter (2) zum zweiten Behälter (20) verhindert.

3. Vorrichtung gemäß Anspruch 1 oder 2, dadurch gekennzeichnet, daß das äußerste Ende des Luftauslasses des Luftrückführungsschlauchs (7) mit dem Hauptschlauch (4, 8, 13, 18, 19) mit einem zwischen diesem Ende des Hauptschlauchs (4, 8, 13, 18, 19) gelegenen Anfangsverbindungspunkt (6) verbunden ist, welcher an den ersten Behälter (2) und den zumindest einen Filter (12) angeschlossen werden kann.

4. Vorrichtung gemaß einem oder mehreren der vorhergehenden Ansprüche, dadurch gekennzeichnet, daß das äußerste Ende des Lufteinlasses des Luftrückführungsschlauchs (7) an den Hauptschlauch (4, 8, 13, 18, 19) an einem zweiten Verbindungspunkt (17) angeschlossen ist, der zwischen dem zumindest einen Filter (12) und dem zweiten Behälter (20) liegt.

5. Vorrichtung gemäß einem oder mehreren der vorhergehenden Ansprüche, dadurch gekennzeichnet, daß sie eine Schließvorrichtung (18a) zum Absperren des Flüssigkeitsstroms im Hauptschlauch (4, 8, 13, 18, 19) umfaßt, die zwischen dem zumindest einem Filter (12) und dem anderen Ende des Hauptschlauchs liegt, wobei die Schließvorrichtung (18a) so wirkt, daß der Luftdurchgang zum zumindest einen Filter (12) während des Luftdurchtritts vom zweiten Behälter (20) zum ersten Behälter (2) verhindert wird.

6. Vorrichtung gemäß Anspruch 5, dadurch gekennzeichnet, daß die Schließvorrichtung (18a) zwischen dem zumindest einen Filter (12) und dem zweiten Verbindungspunkt (17) liegt.

7. Vorrichtung gemäß einem oder mehreren der vorherigen Ansprüche, dadurch gekennzeichnet, daß der Entleerungsschlauch (14) eine zerbrechliche Einrichtung (14a) umfaßt, welche so wirkt, daß der Flüssigkeitsdurchtritt in den Entleerungsschlauch (14) verhindert wird, und auch, daß der Flüssigkeitsdurchtritt in den Entleerungsschlauch (14) nach Zerbrechen der brechbaren Einrichtung ermöglicht wird.

8. Vorrichtung gemäß einem oder mehreren der vorherigen Ansprüche, dadurch gekennzeichnet, daß sie einen zwischen dem äußersten Ende des Hauptschlauchs (4, 8, 13, 19) und den zumindest einen Filter (12) angeordneten Tropffilter (9) umfaßt, der an den ersten Behälter (2) angeschlossen werden kann, wobei der Tropffilter (9) eine Kammer umfaßt, an deren obere Wand der Entleerungsschlauch (14) und der Hauptschlauch (4, 8, 13, 18, 19) angeschlossen sind, während die untere Wand in Verbindung mit dem zumindest einen Filter (12) steht.

9. Vorrichtung gemäß einem der Ansprüche 3 bis 7, dadurch gekennzeichnet, daß sie einen zwischen dem Anfangsverbindungspunkt (6) und dem zumindest einen Filter (12) liegenden Tropffilter (9) umfaßt, wobei letzterer eine Kammer umfaßt, an deren obere Wand der Entleerungsschlauch (14) und der Hauptschlauch (4, 8, 13, 18, 19) angeschlossen sind, wobei die untere Wand mit dem zumindest einen Filter (12) in Verbindung steht.

## Revendications

1. Appareil stérile jetable pour la filtration du sang avec un système permettant d'optimiser la récupération du sang comprenant:
un tube principal (4, 8, 13, 18, 19) contenant de l'air stérile, dont une extrémité peut être reliée à un premier conteneur (2) contenant le liquide à filtrer et dont l'autre peut être reliée à un second conteneur vide (20),
au moins un filtre (12) disposé le long dudit tube principal (4, 8, 13, 18, 19), ledit filtre comprenant un corps de filtre définissant une chambre intérieure et un matériel de filtration (22) divisant l'intérieur de la chambre en deux demi-chambres (21a, 21b), la première demi-chambre (21a) ayant une entrée (11) pour l'entrée du liquide à filtrer et la seconde chambre (21b) ayant une sortie (16) pour évacuer le liquide filtré et
au moins un tube (7) de retour d'air pour le retour de l'air à partir dudit second conteneur (20) vers ledit premier conteneur (2) agissant pour égaliser la pression du liquide aux deux extrémités dudit tube principal (4, 8, 13, 18, 19) et dans ledit au moins un filtre (12) après avoir filtré le liquide,
**caractérisé en ce que**
ladite seconde demi-chambre (21b) dudit au moins un filtre (12) comprend une entrée d'air (15) communiquant avec ladite une extrémité du tube principal (4, 8, 13, 18, 19) au moyen d'un tube d'évacuation (14) agissant pour ventiler ladite seconde demi-chambre après filtration.

2. Appareil selon la revendication 1, caractérisé en que le ledit tube (7) de retour d'air comprend un clapet de non-retour (7a) qui permet le passage de fluide dudit second conteneur (20) vers ledit premier conteneur (2) et empêche le passage de fluide dudit premier conteneur (2) vers ledit second conteneur (20).

3. Appareil selon la revendication 1 ou 2, caractérisé en ce que l'extrémité de sortie d'air dudit tube (7) de retour d'air est reliée audit tube principal (4, 8, 13, 18, 19) au niveau d'un point de connexions (6) initial interposé entre ladite extrémité dudit tube principal (4, 8, 13, 18, 19) qui peut être reliée audit premier conteneur (2) et ledit au moins un filtre (12).

4. Appareil selon l'une quelconque des revendications précédentes, caractérisé en ce que l'extrémité d'entrée d'air dudit tube (7) de retour d'air est reliée audit tube principal (4, 8, 13, 18, 19) au niveau d'un second point (17) de connexions interposé entre ledit au moins un filtre (12) et ledit second conteneur (20).

5. Appareil selon une ou plusieurs des revendications précédentes, caractérisé en ce qu'il comprend un moyen de fermeture (18a) pour couper l'écoulement de liquide dans ledit tube principal (4, 8, 13, 18, 19) interposé entre ledit au moins un filtre (12) et ladite autre extrémité du tube principal, ledit moyen de fermeture (18a) agissant pour empêcher le passage de l'air vers ledit au moins un filtre (12) au cours du passage de l'air provenant dudit second conteneur (20) vers ledit premier conteneur (2).

6. Appareil selon la revendication 5, caractérisé en ce que ledit moyen de fermeture (18a) est interposé entre ledit au moins un filtre (12) et ledit second point (17) de connexion.

7. Appareil selon une ou plusieurs des revendications précédentes, caractérisé en ce que le tube d'évacuation (14) comprend un dispositif fragile (14a) agissant pour empêcher le passage du fluide dans ledit tube d'évacuation (14) et agissant également pour permettre le passage du fluide dans ledit tube d'évacuation (14) après avoir rompu le dispositif fragile.

8. Appareil selon l'une ou plusieurs des revendications précédentes, caractérisé en ce qu'il comprend un filtre de goutte-à-goutte (9), interposé entre ladite extrémité dudit tube principal (4, 8, 13, 18, 19) qui peut être reliée audit premier conteneur (2) et ledit au moins un filtre (12), ledit filtre de goutte-à-goutte (9) comprenant une chambre vers la paroi supérieure où est relié ledit tube (14) d'évacuation et ledit tube principal (4, 8, 13, 18, 19), la paroi inférieure étant en communication avec ledit au moins un filtre (12).

9. Appareil selon l'une des revendications 3 à 7, caractérisé en ce qu'il comprend un filtre de goutte-à-goutte (9) entre ledit point (6) initial de connexion et ledit au moins un filtre (12), ledit filtre de goutte-à-goutte (9) comprenant une chambre vers la paroi supérieure où est reliée ledit tube (14) d'évacuation et ledit tube principal (4, 8, 13, 18, 19), la paroi inférieure étant en communication avec ledit au moins un filtre (12).
